# EUROPEAN PATENT SPECIFICATION

(11) **EP 0 587 716 B1**
(45) Date of publication and mention of the grant of the patent: **16.04.1997**
(21) Application number: 92912508.6
(22) Date of filing: 14.05.1992
(51) Int. Cl.: C07C 5/22, C07C 5/25, C07C 13/43, B01J 23/04, B01J 37/14

(54) **Catalyst and its use for the isomerisation of olefins**
Katalysator und seine Verwendung zur Isomerisierung von Olefinen
Catalyseur et son utilisation pour l'isomérisation d'oléfines

(30) Priority: 14.05.1991 US 700016; 11.10.1991 US 775776
(43) Date of publication of application: 23.03.1994
(73) Proprietor: EXXON CHEMICAL PATENTS INC., Linden, New Jersey 07036-0710 (US)
(72) Inventor: SMITH, Robert, Scott, Houston, TX 77062 (US)
(74) Representative: Bawden, Peter Charles
(86) International application number: US9204017
(87) International publication number: WO9220639

(56) References cited:
- EP-A- 0 230 025
- US-A- 3 405 196
- US-A- 3 897 509
- US-A- 4 675 307

## Description

### 1. Field of the Invention

The present invention relates to a process for isomerizing olefins, and particularly for isomerizing alkenyl bridged ring compounds to the corresponding alkylidene bridged ring compound by use of a highly active and highly selective isomerization catalyst. The process of the invention may be used to isomerize 5-vinyl-2-norbornene (hereinafter "VNB") to 5-ethylidene-2-norbornene (hereinafter "ENB"), which is used commercially in the production of elastomeric polymers and synthetic rubber. The process of the invention may also be used to isomerize a terminal olefin to an internal olefin which may be used as a reactant in the synthesis of chemicals or as an alkylation feedstock to yield octane enhancing gasoline additives. The highly active isomerization catalyst is prepared by adding an alkali metal to alumina and then activating the mixture by careful oxidation of the alkali metal.

The present invention also relates to an isomerization catalyst wherein the alumina has a surface area ranging from 150 to 180 m²/g, and to a catalyst which has been activated at a temperature above 55°C.

### 2. Description of the Prior Art

The isomerization of olefins is well-known. Olefins are often isomerized to produce the type of olefin necessary for a chemical synthesis or for a process for making fuels or fuel additives. For instance, the use of ENB as a monomer in the production of rubbery polymers is well known. ENB may be produced by reacting 1,3-butadiene and cyclopentadiene in an addition reaction commonly known as a Diels-Alder reaction, yielding VNB which is then catalytically isomerized to ENB.

Known isomerization catalysts include liquid bases, such as mixtures of aikali metal hydroxides and aprotic organic solvents, mixtures of alkali metal amides and amines, and mixtures of organic alkali metal compounds and aliphatic amines. Unfortunately, the catalytic activity of the liquid bases is relatively low, and therefore a large amount of these expensive catalysts must be used. Also, recovery of the catalyst from the reaction mixture is very difficult, requires complicated separation and recovery steps and consumes a large amount of energy.

Solid isomerization catalysts are also known, for example, alkali metals carried on large surface area anhydrous supports such as activated carbon, silica gel, alumina and the like. These solid catalysts are difficult to handle because they may ignite and lose activity on contact with oxygen. Also, the isomerization performance of these solid catalysts is poor, because conversion and selectivity are low.

U.S. Patent 3,897,509 discloses that heating an alkali metal, an alkali metal hydroxide and alumina yields an alkali catalyst composition which is described to be stable on exposure to air and water and active in various chemical reactions. Particularly the catalyst is said to be useful in the isomerization of alkenyl bridged ring compounds to the corresponding alkylidene bridged ring compounds. Thus, it is useful for the production of alkylidene bridged ring compounds such as ENB which, as mentioned above, is valuable in the production of synthetic rubber.

The preferred method of forming the catalyst of U.S. 3,897,509 is by heating and mixing an alkali metal, an alkali metal hydroxide and alumina at a temperature higher than the melting point of the alkali metal; however, the catalyst may also be prepared without the use of an alkali metal hydroxide if the starting alumina contains water.

U.S. Patent 3,405,196 discloses a process in which a terminal olefin is converted to an internal olefin in the presence of a supported alkali-metal catalyst that has been pretreated with an oxygen containing gas such as nitrous oxide. The catalyst used contains an alkali metal dispersed on a high-surface area, substantially inert support. The alkali metal may be selected from sodium, potassium, rubidium and cesium. The desired catalyst support material is a high surface area, large pore, and slightly acidic alumina. The catalyst is primarily used to convert 1-pentene to 2-pentene and 1-butene to 2-butene and is not used to isomerize an alkenyl bridged ring compound to an alkylidene bridged ring compound.

US-A-4675307 discloses alkali-metal-supported catalysts for use in the isomerisation of olefins in a calcination product of a hydrotalcite compound is treated with molecular oxygen to convert part of the metal to oxide to give a more stable substance.

The prior art catalysts are either explosive or lack desirably high activities. These and other disadvantages of the prior art are overcome by the present invention, which provides a new selective and efficient catalyst for isomerization of olefins.

### SUMMARY OF THE INVENTION

The present invention relates to a process and catalyst for isomerizing olefins, and particularly to isomerizing alkenyl bridged ring compounds to the corresponding alkylidene bridged ring compounds by use of a highly active and highly selective isomerization catalyst. In a preferred embodiment, the process of the invention is used for isomerizing 5-vinyl-2-norbornene an alkenyl bridged ring compound, to 5-ethylidene-2-norbornene, an alkylidene bridged ring compound, which is used commercially as the diene monomer in ethylene-propylene-diene monomer rubber (EPDM). The highly active isomerization catalyst is prepared by adding an alkali metal to a properly prepared calcined support material followed by careful oxidation of the alkali metal. The calcined support material has a surface area of from 150 to 180 m²/g.

The process for isomerizing an alkenyl bridged ring compound to the corresponding alkylidene bridged ring compound comprises contacting the alkenyl bridged ring compound with an isomerization catalyst comprising an alkali metal dispersed on a calcined support wherein the isomerization catalyst is treated with an oxygen containing gas before contact with the alkenyl bridged ring compound. One particular embodiment of the process for catalytically isomerizing a chemical plant stream comprises contacting the stream comprising an alkenyl bridged ring compound with an activated catalyst. The activated catalyst is prepared by heating a support material other than hydrotalcite to form a calcined support material having a surface area of 150 to 180 m²/g contacting the calcined support material with an alkali metal to form a catalyst precursor mixture of alkali metal and calcined support material, and contacting the catalyst precursor mixture with an oxygen containing activating gas.

Another embodiment of the invention provides a catalyst which has been activated at a higher temperature than is known in the art. The activated catalyst is prepared by:
(a) heating a support material other than hydrotalcite to form a calcined support material having a surface area of 150 to 180 m²/g;
(b) contacting the calcined support material with a metallic form alkali metal to disperse the alkali metal on the support material and form a catalyst precursor mixture of alkali metal and calcined support material; and
(c) contacting the catalyst precursor mixture with an oxygen containing activating gas.

A feature of this invention is the high level of VNB conversion and the high selectivity to ENB. Another feature of this invention is the stability of the catalyst used to effect isomerization. An object of the invention is to provide an isomerization catalyst which combines high selectivity and conversion efficiency and yet remains non-explosive on contact with air or water. Accordingly, these and other features of this invention will become apparent from the following detailed description, wherein reference is made to the Figures in the accompanying drawings.

### IN THE DRAWINGS

Figure 1 is an X-ray diffraction pattern for alumina.

Figure 2 is an X-ray diffraction pattern for a catalyst made according to the invention.

Figure 3 is an X-ray diffraction pattern for a catalyst made according to the prior art.

Figure 4 is a graph showing the relationship between catalyst activity and the surface area of the support.

Figure 5 is a graph showing the relationship between the amount of oxygen used to activate the catalyst and the activity of the catalyst.

### DETAILED DESCRIPTION OF THE INVENTION

The isomerization of olefins described herein relates to the movement of the olefinic double bond from its initial position in the olefinic molecule. It is often necessary to change the location of the double bond in order to provide the proper reactant for a desired chemical synthesis. For instance, linear alpha olefins such as 1-butene, 1-pentene, 1-hexene, 1-heptene, and 1-octene may be isomerized to yield internal olefins such as 2-butene, 2-pentene, 2-hexene, 3-hexene, 2-heptene, 3-heptene, 2-octene, 3-octene and 4-octene. Additionally, branched alpha olefins such as 2-methyl-1-butene and 3-methyl-1-pentene may be isomerized to 2-methyl-2-butene and 3-methyl-2-pentene, respectively. Internal olefins and branched internal olefins may also be isomerized according to the invention which may be used to isomerize any olefin with three or more carbon atoms.

The invention is particularly useful in the production of ENB from VNB which may be produced by a Diels-Alder reaction. The Diels-Alder reaction is an additive reaction of an olefin with a conjugated diene. The Diels-Alder reaction proceeds without a catalyst at temperatures ranging from about 50° to 250°C. Alkenyl bridged ring compounds may be produced by the Diels-Alder reaction of cyclodienes with aliphatic dienes. VNB is produced by the Diels-Alder reaction of 1,3-butadiene with cyclopentadiene. The conditions necessary to bring about the Diels-Alder reaction of 1,3-butadiene with cyclopentadiene are well known in the art. In particular, butadiene may be contacted with cyclopentadiene in the liquid phase at temperatures of from 100° to 200°C and a pressure of from 150 to 300 Ibs/square inch. It is not necessary to use a catalyst to advance the Diels-Alder reaction. The reaction is generally completed in 0.1 to 100 hours and is usually conducted under an inert atmosphere. Preferably, the reaction may be conducted in a liquid state, most preferably in a liquid-full reaction vessel.

The preferred reaction occurs between 1,3-butadiene and cyclopentadiene, however, undesirable polymerization reactions may also occur. For example, 1-3-butadiene monomers may react with other 1,3-butadiene monomers to form polymers. Similar polymerization reactions may occur between cyclopentadiene monomers, and between 1,3-butadiene and cyclopentadiene. Certain compounds are known which suppress or inhibit the undesired polymerization reactions. Any one or combination of these inhibitor compounds may be added to the reactants in order to produce more VNB from the same amount of starting material and to avoid plugging certain parts of the reaction apparatus with the high molecular weight polymers which might otherwise be formed. Many inhibitor compounds are known in the art including 2,6-di-t-butyl-p-cresol, diphenylnitrosamine, and N-substituted p-phenylenediamines.

The reaction product mixture may be passed directly to the isomerization process, however, it is preferred to purify the reaction product in order to yield a stream predominately containing VNB. The reaction product stream may be purified by fractional distillation under increased pressure, atmospheric pressure or preferably at reduced pressures. Extractive distillation techniques may also be used and some of the known extractive distillation solvents are acetonitrile, water, sulfolane and N-methylpyrrolidone (NMP). The reaction product may also be purified by extraction, extraction followed by distillation, or crystallization.

The Diels-Alder reaction product or preferably the purified VNB is then isomerized in order to convert VNB to ENB. The VNB stream to be isomerized may be 90% or more by volume VNB or the VNB stream may be diluted with a solvent that is inert during the isomerization process. Appropriate solvents include aliphatic compounds such as hexane, heptane, octane and isooctane, and aromatic compounds such as benzene, toluene, xylene and ethylbenzene. Many other solvents are acceptable and within the scope of the invention: however, certain types of compounds are isomerization catalyst poisons and should not be used as solvents. These poisons include water, alcohols, acid compounds and conjugated diene compounds.

The isomerization catalyst of the invention is comprised of an alkali metal on a calcined support material, the combination being further treated with a gas containing oxygen. The alkali metal may be selected from the group of lithium, sodium, potassium, rubidium, cesium and mixtures thereof. The alkali metal consists essentially of the metal in its elemental state, for example, if potassium is the alkali metal, it should be added as pure potassium and not in combination with another element, i.e. potassium hydroxide. Two or more alkali metals may be combined in the same catalyst. Sodium is the preferred alkali metal. The catalyst may be prepared by dispersing 1-40 wt % alkali metal on a calcined support material at temperatures of from 100-600°C under an atmosphere of dry, inert gas. The alkali metal dispersed on the support material is then subjected to oxidation by contact with a gas having an oxygen content of 0.1 to 25 mole % at temperatures of 0-300°C.

The material of the support upon which the alkali metal should be dispersed may be any normally solid material which has large pores and a surface area ranging from 150 to 180 m²/g after calcination, but the support material may not be a hydrotalcite compound as described in U.S. Patent 4,675,307. The support material should remain in the solid state at the elevated temperatures required during calcination and alkali metal dispersion. Some surprisingly active catalysts have been prepared from support material with a surface area of about 170 m²/g. Catalysts have been prepared from support material with a surface area of about 170 m²/g. Catalysts with a surface area of 180 m²/g are also good. Calcination, as described herein, at temperatures below 400°C does not significantly change the surface area of the support. The surface area of the alumina is measured by the BET (Brunauer, Emmett, Teller) method of nitrogen adsorption and desorption.

The pore size of the support material may range from 5 to 1.000 Angstroms more preferably from 10 to 300 Angstroms and most preferably from 10 to 100 Angstroms. The support material used for preparing the catalyst may be in powder, pelletized, or extruded form. The support material should be calcined under a dry atmosphere, and it should be free of water and entrained oxygen after calcination.

Alumina, silica, and oxides of metals of Groups 3A, 4A and 4B of the Periodic Table may be used as the support material. The Periodic Table referred to is the table as reproduced in the CRC Handbook of Chemistry and Physics, 53rd edition. Other examples of specific support materials include carbon, graphite, magnesia, titania, zirconia, calcium oxide, magnesium oxide, sodium oxide and barium oxide. Alumina is the preferred support material and in a most preferred embodiment, the support material consists essentially of alumina. Alumina consists of mostly Al₂O₃ and small amounts of Na₂O, SiO₂ and Fe₂O₃. The form of alumina utilized in the most preferred embodiment is gamma alumina.

The support should be calcined prior to contact with the alkali metal. Calcination is effected by heating the support material at temperatures of from 100° to 1,000°C, more preferably from 200° to 800°C and most preferably from 200° to 400°C. The calcination may be conducted at atmospheric pressure or higher, however, reduced pressures are preferred. Preferably an inert gas is purged through the calcination vessel in order to sweep away any water or oxygen molecules which may be driven off from the support material by the heat. The calcination should last for from 0.1 to 100 hours preferably from 1 to 20 hours. The heat of calcination drives water from the support material, and provides an essentially dry support material. After calcination, the support material should be kept under a dry atmosphere so that water does not become associated with the support material.

As mentioned above, calcination at temperatures below 400°C for less than 20 hours does not significantly change the surface area of alumina. However, calcination at temperatures above 500°C may result in a decrease in the alumina surface area. In fact, high temperature calcination may be used to reduce the surface area of aluminas from values above 195 m²/g to values within the desired range. For example, an alumina with a surface area of 240 m²/g may be calcined at 600°C for 1 to 2 days to yield an alumina with a surface area of 170 m²/g.

The catalytic combination of alkali metal and support material is prepared by first contacting the calcined support material with the alkali metal under a dry, inert, oxygen free atmosphere thus forming a catalyst precursor mixture. The alkali metal is preferably in the molten state during at least a portion of the contact and the contact occurs by physically blending the calcined support material with the alkali metal under the dry, inert, oxygen free atmosphere. In the most preferred embodiment, the catalyst precursor mixture consists essentially of the alkali metal and the support material.

The alkali metal may be in the solid, liquid or gaseous state when initially contacted with the calcined support material. Usually, the initial contact occurs at a temperature of from 0°C to 500°C, preferably from 10°C to 40°C under an inert atmosphere. Gases such as nitrogen, argon, helium and krypton will provide an inert atmosphere. If the alkali metal is in the solid state when initially contacted with the support material, the temperature of the mixture should be raised enough to melt the alkali metal, and the mixture should be stirred for initial dispersion of the alkali metal on the support material. The resulting mixture is then heated to a temperature of from 100°C to 600°C, preferably 100°C to 300°C, more preferably 120°C to 200°C under constant agitation.

The mixing agitation may be discontinued after the alkali metal is roughly dispersed on the support material, however, it is preferable to continue mixing until the alkali metal is evenly distributed on the surface of the calcined support material. In the case of a mixture of sodium on alumina, a visual indication of a blue black or blue gray evenly distributed color over the surface of the alumina indicates uniform distribution of sodium. If large amounts of materials are to be mixed or slow mixing speeds are used, complete uniform dispersion will take longer, however, mixing is often complete within about two hours when a rotary mixer is used to produce 100 grams of catalyst.

The amount of alkali metal which should be distributed upon the calcined support material is in the range of from 1 to 40 wt% based upon the total weight of the mixture comprising both alkali metal and calcined support material. Preferably the range of alkali metal is from 5 to 20 wt% and most preferably from 10 to 15 wt%.

After the alkali metal has been dispersed upon the calcined support material to form a catalyst precursor mixture, the mixture should be contacted with an activating gas containing oxygen. Since a violent reaction between the alkali metal and oxygen will occur if oxygen is added too quickly, care should be taken to avoid exposing the mixture to excessive oxygen initially. If the temperature of the mixture exceeds about 300°C during the activation, the rate of oxygen contact with the mixture should be reduced. The rate of oxygen contact may be reduced by lowering the flow rate of oxygen containing gas or by reducing the concentration of oxygen in the oxygen containing gas. Some inert gas flow should be maintained in order to reduce the temperature of the catalyst mixture to a safe level. Alternatively, pure oxygen at reduced pressure may be used as the activating gas.

In one embodiment of the invention, the catalyst mixture is contacted with the activating gas at a temperature within the range of from 0° to 300°C preferably 20° to 150°C and most preferably 25 to 50°C. The catalyst mixture may be agitated while the contact with the activating gas occurs. In another embodiment of the invention, the catalyst mixture is contacted with the activating gas at high temperatures. Typically, the temperatures should be 55° to 300°C, preferably 65° to 300°C and most preferably 100° to 300°C.

The activating gas may comprise a single type of gaseous compound containing oxygen or a mixture of an inert gas with another gas containing oxygen. Examples of activating gases include nitric oxide, nitrous oxide, sulfur dioxide, dry air, ozone, and mixtures of oxygen with nitrogen, helium, argon, krypton, xenon, or radon. Mixtures of these gases or mixtures may also be used. In a preferred embodiment a mixture of oxygen (O₂) in nitrogen (N₂) may be used as the activating gas. The amount of oxygen in the activating gas should be from 0.1 to 25 mole %. Preferably the amount of oxygen in the activating gas mixture is from 2 to 10 mole % and most preferably 5 mole %. If pure oxygen (100 mole % O₂) is used as the activating gas, the pressure should be reduced to between 0.001 and 0.25 atmosphere.

If oxygen is only part of an oxygen containing molecule, such as NO or NO₂, the oxygen content of the gas is calculated by dividing the atomic weight of oxygen in the molecule by the molecular weight of the gas.

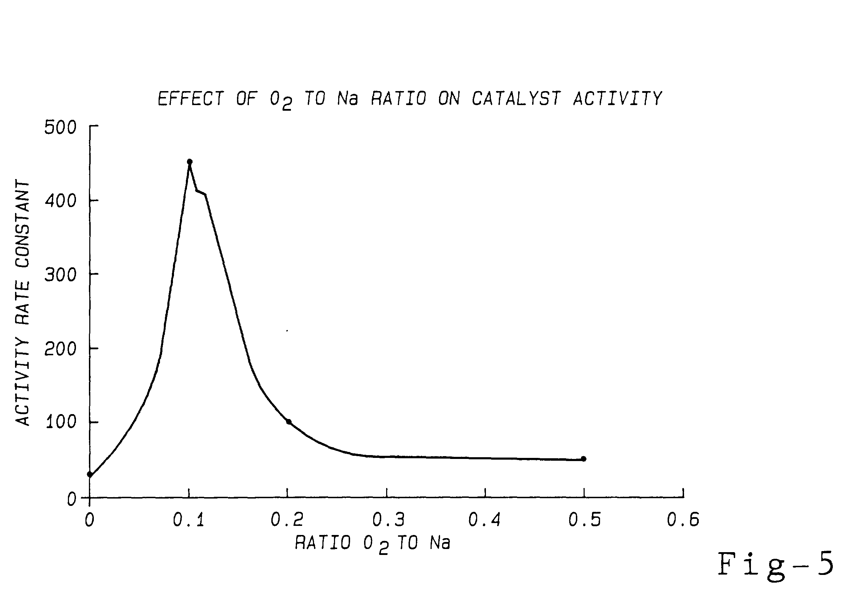

The contact between the oxygen containing gas and the mixture may be continued until all of the alkali metal has been oxidized; however, it is preferable to stop the contact when the total contact oxygen to alkali metal atomic ratio is within the range of from 0.005 to 1.0 moles of oxygen per atom of alkali metal. Preferably the ratio is within the range of from 0.01 to 0.2, more preferably 0.03 to 0.18 and most preferably 0.05 to 0.15 moles of oxygen per atom of alkali metal. These ranges reflect the total moles of oxygen contacted with the alkali metal. At these ranges most, usually all, of the oxygen contacted with the alkali metal reacts with the alkali metal. This is apparent in Figure 5 where contact of more than 0.5 moles of oxygen per atom of sodium does not result in further change in catalyst activity.

The catalyst prepared as described above is then used to isomerize olefins. For example, the catalyst may be used to isomerize alkenyl bridged ring compounds, such as VNB, to alkylidene bridged ring compounds, such as ENB. Alkenyl bridged ring compounds are of the general formula (I): 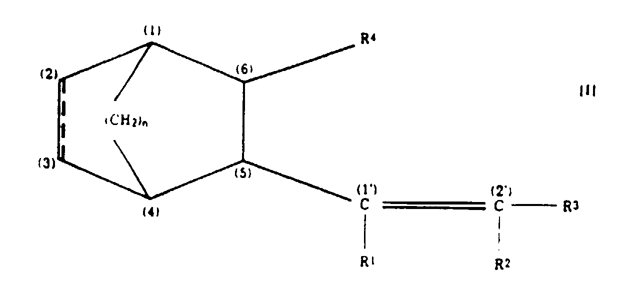 wherein R¹,R² and R³ are each hydrogen or alkyl having 1 to 8 carbon atoms, R⁴ is hydrogen or alkyl having 1 to 4 carbon atoms, and n is 1 or 2 and wherein a double bond may be present at the place between the 2- and 3-positions as indicated by the dotted line.

Specific examples of some alkenyl bridged ring compounds are:
5-vinylbicyclo[2.2.1]heptane;
5-(1'-propenyl)-bicyclo[2.2,1]heptane;
5-(1'-butenyl)-bicyclo[2,2,1]heptane;
5-isopropenylbicyclo[2.2,1]heptane;
5-(2'-methyl-1'-propenyl)bicyclo[2,2,1]heptane;
6-methyl-5vinylbicyclo[2,2,1]heptane;
6-methyl-5-isopropenyl[bicyclo[2,2,1]heptane:
6-ethyl-5-vinylbicyclo[2,2,1]heptane;
5-vinylbicyclo[2,2,1]hepta-2-ene;
5-(1'-propenyl)-bicyclo[2,2,1]hepta-2-ene;
5-(1'-butenyl)-bicyclo[2,2,1]hepta-2-ene;
5-isopropenylbicyclo[2,2,1]hepta-2-ene;
5-(2'-methyl-1-propenyl)-bicyclo[2,2,1]hepta-2-ene;
5-(1'-octenyl)-bicyclo[2,1,1]hepta-2-ene;
6-methyl-5-vinylbicyclo[2,2,1]hepta-2-ene;
6-methyl-5-isopropenylbicyclo[2,2,1]hepta-2-ene;
6-ethyl-5-vinylbicyclo[2,2,1]hepta-2-ene;
5-vinylbicyclo[2,2,1]octane;
5-(1'-propenyl)-bicyclo[2,2,2]octane;
5-isopropenylbicyclo[2,2,2]octane;
5-(1'-butenyl)-bicyclo[2,2,2]octane;
6-methyl-5-vinylbicyclo[2,2,2]octane;
6-methyl-5-isopropenylbicyclo[2,2,2]octane;
5-vinylbicyclo[2,2,2]octane;
5-(1'-propenyl)-bicyclo[2,2,2]octa-2-ene;
5-isopropenylbicyclo[2.2,2]octa-2-ene;
5-(1'-butenyl)-bicyclo[2,2,2]octa-2-ene;
5-(2-methyl-1'-propenyl)-bicyclo[2,2,2]octa-2-ene;
6-methyl-5-vinylbicyclo[2,2,2]octa-2-ene; and
6-methyl-5-isopropenylbicyclo[2.2.2]octa-2-ene.

The compounds represented by formula I can be produced by subjecting a cyclic diene such as cyclopentadiene or cyclohexadiene and an aliphatic 1.3-diene to a Diels-Alder reaction or by subjecting the corresponding bridged ring compound bearing a hydroxyl group at the 1'- or 2'-position to dehydration.

Isomerization of an alkenyl bridged ring compound shifts the double bond from 1'-2' position to the 5-1' position of formula I thereby forming the alkylidene bridged ring compound. The isomerization is effected by contacting an alkenyl bridged ring compound, for example, a VNB stream, with the catalyst at temperatures of from -50° to 200°C. Preferred temperatures of isomerization range from 0° to 150°C, most preferably from 20° to 100°C; however, a temperature of at least 80°, preferably about 100°C provides unique advantages if the VNB is contaminated with catalyst poisons such as cyclopentadiene. The high temperature isomerization embodiment of the invention is particularly useful for isomerizing chemical plant streams comprising a substantial amount of alkenyl bridged ring compounds, but further comprising isomerization catalyst poisoning compounds. This is because of the surprising and unexpected discovery that this particular catalyst is resistant to catalyst poisons. The catalyst is especially effective at higher temperature isomerization conditions.

The amount of catalyst used is not critical although the use of more catalyst will reduce the time necessary to bring about a given level of conversion at the same temperature. Generally, the weight of alkali metal utilized per weight of alkenyl bridged ring compound ranges from 1 part alkali metal per 10,000 parts of alkenyl bridged ring compound to 1 part alkali metal per 500 parts of alkenyl bridged ring compound. In fixed bed processes, the weight average space velocity expressed in terms of weight of feed per hour divided by the weight of the supported catalyst in the fixed bed should range between 0.1 to 500 Hr⁻¹.

The isomerization process may be conducted at any pressure, however, pressures of from about 0 to about 100 psig are preferred. The reaction may be conducted in liquid phase or gas phase. Reaction time varies depending on the reaction temperature, and the amount of catalyst used, but generally ranges from 5 minutes to 6 hours. The isomerization reaction is generally conducted in the absence of oxygen and water.

Use of the above described catalyst of the invention in the isomerization of VNB to ENB usually results in nearly complete conversion to ENB and therefore no product purification is necessary. The resulting ENB may be transferred directly to a polymerization process to make EPDM rubber. If for some reason the conversion rates are lower, the ENB may be purified of VNB by distillation.

Although the isomerization of VNB to ENB has been described in more detail, the catalyst is also useful in the isomerization of olefins in general in the same way as described in reference to VNB isomerization. Conversion percentages herein are determined by subtracting the weight amount of compound remaining after isomerization from the beginning amount of the compound and dividing the result by the beginning amount of the compound. The resulting quotient is multiplied by 100% to express the term in percent. Selectivity is determined by dividing the amount of material converted into desired product by the total amount of material converted and multiplying the result by 100%.

Particular aspects of the invention may be further understood by reference to the examples below.

### Example 1

This example demonstrates a method of preparing a catalyst according to the invention. The following procedures were conducted in a N₂ dry-box. Fifteen grams of calcined, 50/100 mesh alumina powder and 1.5g of fresh-cut sodium (65.2 mg-atom) were placed in a 300 mL 3-neck flask equipped with a stir shaft with a glass paddle, a "Y"-adapter, a hose nipple, a gas addition tube, and a stopper. The flask was subjected to a vacuum for 30 minutes, and then the flask was filled with nitrogen. The flask was taken from the dry-box, and filled with dry nitrogen. The contents were stirred, and heated with an electric heating mantle to a skin temperature of 400°C for 2 hours to form an evenly dispersed mixture of sodium on alumina. Afterwards, the skin temperature was reduced to 150°C, and an activating gas of 5% O₂ and 95% N₂ was added at 0.07 L/min via the gas addition tube. The activating gas addition continued for 90 min with good stirring. The resulting catalyst was then allowed to cool to room temperature under a N₂ atmosphere.

Two grams of the catalyst prepared as noted above were placed all at once into a vessel containing water. There was no indication of gas evolution or any strong exothermic reaction.

### Example 2

This example demonstrates isomerization of VNB to ENB at low temperatures according to one embodiment of the invention. In a N₂ dry-box, 25g of VNB (208 mmole) and 2g decane were placed into a 50 mL Erlenmeyer flask equipped with a teflon coated spin-bar. Then, 0.5g of the catalyst prepared according to Example 1 was added, and the mixture was stirred vigorously at room temperature. After 3 hours, the VNB conversion was 99.6% and the selectivity to ENB was 99.7%.

### Example 3

This example demonstrates isomerization of VNB to ENB at relatively high temperatures according to one embodiment of the invention. Under nitrogen, 50g of dry VNB (417 mmole) and 5g of decane, as a GC standard, were placed into a 300 mL round-bottomed flask equipped with a paddle stirrer. The VNB mixture was heated to 120°C, and 1.0g of catalyst prepared according to Example 1 was added while vigorous stirring was maintained. After 60 minutes, the VNB conversion was 98%, and the selectivity to ENB was 99.7%.

### Example 4 (Comparative)

This example illustrates a high temperature activation of the catalyst. Fifteen grams of alumina powder with a surface area of 90 m²/g was calcined for one hour at 500°C under a nitrogen atmosphere. The calcined alumina was then cooled to room temperature and subjected to a vacuum for 30 minutes and thereafter blanketed with nitrogen, 1.5g of sodium was added to the calcined alumina, and the mixture was heated at a rate of 6°C/min with stirring until a temperature of 400°C was reached. The stirring of the mixture continued while the temperature of the mixture was held at 400°C for one hour. After the mixture cooled to a temperature of 150°C, an activating gas comprising 5% 0₂ and 95% N₂ by volume was passed through the mixture for 90 minutes at a rate of 0.07L/min.

### Example 5 (Comparative)

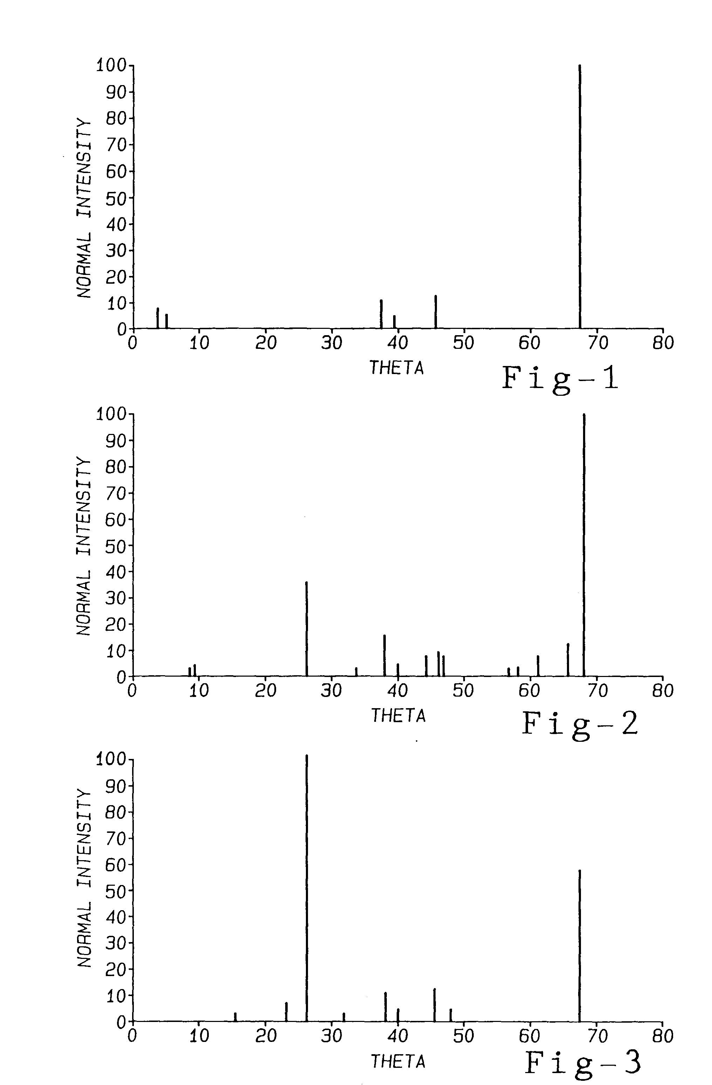

X-ray diffraction patterns for three solids were measured and are compared in the Figures 1-3. The X-ray diffraction pattern for the raw alumina from Example 4 is represented by Figure 1. The X-ray diffraction pattern for the sodium catalyst made according to Example 4 is represented at Figure 2. The X-ray diffraction pattern for a catalyst made according to U.S. Patent 3,928,485 using the same alumina used in Example 4 is represented by Figure 3. The catalyst made according to U.S. 3,928,485 was prepared as follows.

Fifteen grams of dried alumina and 1.5g NaOH were ground together, and then mixed under nitrogen for 2 hours at a temperature of 330°C. The temperature was then lowered to 90°C and 0.83g of sodium was added to the mixture. Thereafter the mixture was stirred and heated to a temperature of 400°C. Stirring continued for 2 hours while the temperature was maintained at 400°C.

### Example 6

This example demonstrates isomerization in the presence of an isomerization catalyst poison, 0.25g of a catalyst made according to Example 1 was stirred at 120°C with 25g of VNB which contained 0.03% cyclopentadiene, a known isomerization catalyst poison. After one hour of stirring, 60% of the VNB had been converted and all of the cyclopentadiene had been converted.

4.0g of a catalyst prepared in accordance with Example 1 was stirred at 25°C with 200g VNB which contained 0.06% cyclopentadiene. After one hour of stirring, the cyclopentadiene concentration was reduced to 0.03% and the VNB conversion was 10%. This example shows that the negative effects of cyclopentadiene are less pronounced at higher temperature isomerization. The example also shows that the cyclopentadiene may be removed by the higher temperature isomerization reaction.

### Example 7 (Comparative)

This example demonstrates the extra safety of using an activated catalyst. An alumina with a surface area of 210 m²/g was calcined at 400°C for one hour and then held under a vacuum at 200°C for one hour. 4.5g of sodium metal was added to two separate 30g portions of the calcined alumina. The resulting mixtures were stirred at 210°C for one hour after which the temperature was increased to 410°C and mixing continued for one more hour. The mixtures were then cooled to room temperature. The first portion was contacted with water resulting in a very violent exothermic reaction. The second portion was first treated with an activating gas of 5 volume % O₂ and 95 volume % N₂. The second activated catalyst portion was treated with water in a manner similar to the first portion but only a very mild exothermic reaction resulted.

### Example 8 (Comparative)

This example demonstrates that the activated catalyst has superior resistance to catalyst poisons. The activating gas treated catalyst prepared according to Example 7 was stirred for one hour at 25°C in a solution of VNB with 0.06% cyclopentadiene. The amount of catalyst used was 4 wt% of the total weight of the solution and the conversion of VNB was 40%. When the same amount of catalyst prepared according to Example 7 without the activating gas treatment was similarly contacted with the same solution of VNB with cyclopentadiene, the conversion of VNB was 25%.

### Example 9 (Comparative)

This example demonstrates that the activated catalyst has an activity equivalent to a more dangerous non-activated catalyst. The activated and non-activated catalysts prepared in Example 7 were contacted with purified VNB. For each contact, 2 wt% of catalyst was used based on the weight of purified VNB. The conversions after one hour were 52% for the activated catalyst and 58% for the non-activated catalyst.

### Example 10

This example demonstrates the reactivity of sodium on alumina with water. Sodium materials are difficult to handle because of their explosive reactivity with air and water and therefore require special care in shipping and processing. Twenty grams of alumina powder and 3 grams of Na (0.13 gram-atom) were mixed at 300°C for 1 hour under a nitrogen atmosphere. The mixture was cooled, and then carefully mixed with kerosene. Addition of water to this slurry set off an exothermic reaction that was accompanied by evolution of large quantities of gas from the mixture.

### Example 11

This example demonstrates various catalysts prepared on alumina with different surface areas. For each catalyst prepared. the alumina was calcined at 400°C in air for 1 hour. Thereafter, the air was removed and the temperature reduced to 200°C. The alumina was maintained under vacuum at 200°C for 1 hour.

Thirty grams of the alumina prepared as noted above were then mixed with 4.5 g of sodium under a nitrogen atmosphere at 210°C. The mixing continued for 1 hour after which the temperature was increased to 410°C. Mixing continued at 410°C for 1 more hour under the nitrogen atmosphere resulting in an evenly distributed mixture of sodium on alumina.

The mixture was cooled to room temperature (25°C) and then contacted with a gas consisting of 5% oxygen and 95% nitrogen. The gas flow rate was maintained at 126 mUmin while the mixture was continuously stirred for 152 minutes.

VNB was contacted with 2 wt% of the catalyst prepared as noted above. The VNB/catalyst solution was stirred at room temperature for 1 hour. A sample of the resulting solution was taken and the conversion of VNB was measured.

The 11-A alumina was supplied as AL-0171P by Engelhard and had a surface area of 210 m²/g. The VNB conversion for 11-A was 55%. The 11-B alumina was supplied by Engelhard designated AL-1040P and had a surface area of 172 m²/g. The VNB conversion for 11-B was 99.8%. The 11-C alumina was supplied by Engelhard as AL-3916P and had a surface area of 155 m²/g. The conversion level for 11-C was 99.8%.

### Example 12

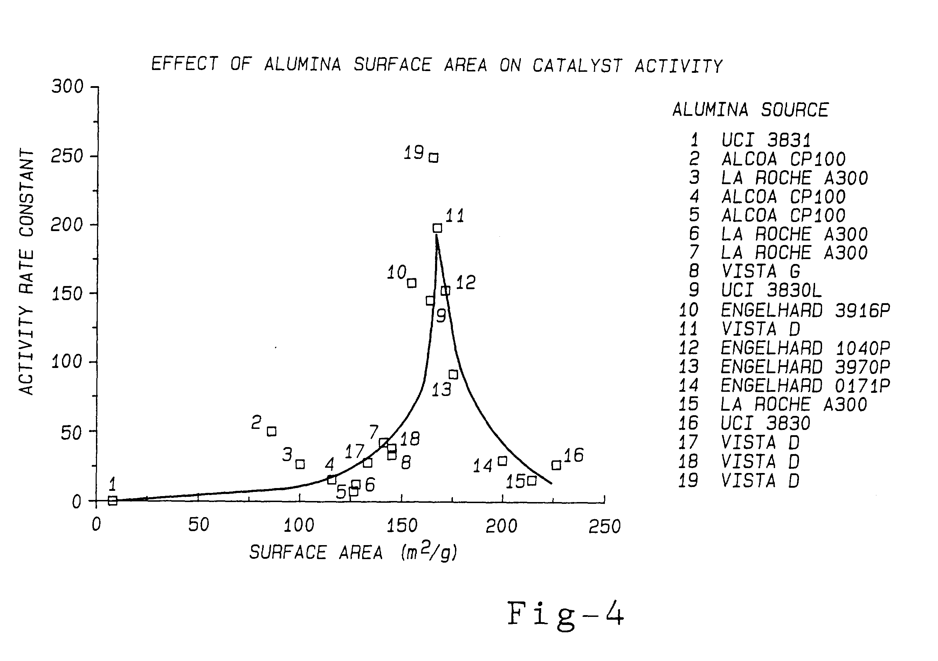

Figure 4 shows the activity rate constant for the isomerization of VNB to ENB at room temperature over catalysts prepared according to the invention. The surface area of the alumina support material used to prepare the catalysts varied from less than 10 square meters per gram to more than 225 m²/g before calcination, as indicated by the horizontal axis of Figure 4. The activity rate constant was calculated according to the following formula: [In(VNBᵢ/VNB_{f})] ö [(catalyst wt% Na)(reaction time in hours)], where VNBᵢ is the initial concentration of VNB, and VNB_{f} is the concentration of VNB at the designated reaction time. The figure clearly shows that when alumina support material is selected to have a surface area within the range of 150-180 m²/g, the activity of the catalyst for this reaction can be significantly improved over activities of catalysts outside of the selected surface area range. The most active catalyst is prepared from alumina with a surface area of about 170 m²/g.

### Example 13 (Comparative)

This example shows the preparation of catalyst from an alumina support with a surface area outside of the selected range. Alumina powder (30g) having a surface area of 200 m²/g was placed in a round bottom flask under a nitrogen blanket at room temperature along with 4.5g sodium metal which had been cut into small pieces. The mixture inside the round bottom flask was stirred and heated to 150°C for 30 minutes. The sodium metal dispersed onto the alumina resulting in a blue-gray powder.

That mixture was then heated to a temperature of 300°C and stirring was continued for 60 minutes. The sodiumialumina mixture was cooled to room temperature after which. a gaseous stream of 5% O₂ in N₂ was passed through the flask at a rate of 126 mL/min until the molar ratio of O₂ to the initial amount of Na was 02:1. Analysis of the catalyst showed that 6 wt% of the initial amount of Na remained unreacted with O₂. The activity rate constant of this catalyst for isomerization of VNB to ENB at room temperature was measured as 31.

### Example 14 (Comparative)

This example describes the preparation of a catalyst from alumina support material that had a surface area outside of the selected range. The catalyst was prepared following the same procedure as outlined in Example 13, except that the alumina had a surface area of 136 m²/g. Analysis of the catalyst showed that 25 wt% of the initial Na remained unoxidized. The activity rate constant of this catalyst for the isomerization of VNB to ENB at room temperature was measured as 52.

### Example 15

This example describes the results achieved using a catalyst prepared from an alumina within the selected range of surface area. The catalyst was prepared according to the same procedure as outlined in Example 13 except that the alumina support material had a surface area of 178 m²/g. Analysis of the catalyst prepared showed that 5 wt% of the initial Na remained unoxidized. The activity rate constant of this catalyst for the isomerization of VNB to ENB at room temperature was 87.

### Example 16

This example shows the preparation and activity of a catalyst within the most preferred range of alumina surface area. The catalyst was prepared according to the same procedure outlined in Example 13 except that the alumina had a surface area of 170 m²/g. Analysis of the resulting catalyst showed that 19 wt% of the initial Na metal remained unoxidized. The activity rate constant of this catalyst for the isomerization of VNB to ENB at room temperature was measured as 158.

### Example 17

This example describes the preparation of a catalyst at a lower maximum dispersion temperature than was used in Examples 12-16. Alumina powder (30g) with a surface area of 178 m²/g, and 4.5g Na metal, cut into pieces, were placed in around bottom flask under a nitrogen atmosphere at room temperature. The mixture was stirred and heated to a temperature of 150°C for 30 minutes. After this step the sodium was dispersed on the alumina yielding a blue-gray powder. The temperature of the mixture was maintained at 150°C for an additional 60 minutes with continuous stirring. After that, the mixture was cooled to room temperature. A stream of nitrogen containing 5 vol% O₂ was passed through the flask at a rate of 126 mL/min until the or molar ratio of O₂ to initial Na was 0.2:1. Analysis of the resulting catalyst showed that 11 wt% of the initial sodium remained unoxidized. The activity rate constant of this catalyst for isomerization of VNB to ENB at room temperature was measured as 189.

The activity rate constant of the catalyst of this Example 17 is much higher than the 87 reported for the same catalyst prepared at a higher mixing temperature of 300°C as in Example 15. This Example 17 indicates that mixing at a lower temperature of 150°C improves the activity of the finished catalyst by a factor of 2.17 compared to a catalyst prepared with mixing at 300°C.

### Example 18

The steps described in Example 17 were repeated except that the mixing temperature was maintained at 220°C instead of 150°C in the second step described above. Analysis of the resulting catalyst showed that 12 wt% of the initial sodium remained unoxidized. The activity rate constant of this catalyst for isomerization of VNB to ENB at room temperature was measured as 285. Comparison of the results from Examples 15, 17 and 18 show that there is an optimum temperature for performing the mixing of sodium with alumina. The following Table 1 indicates the differences obtained.

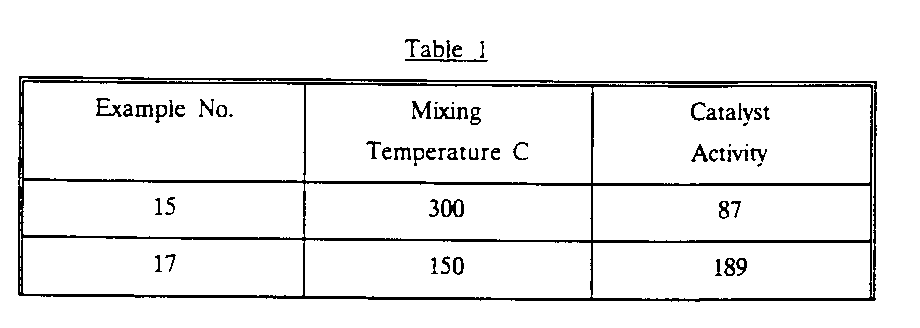

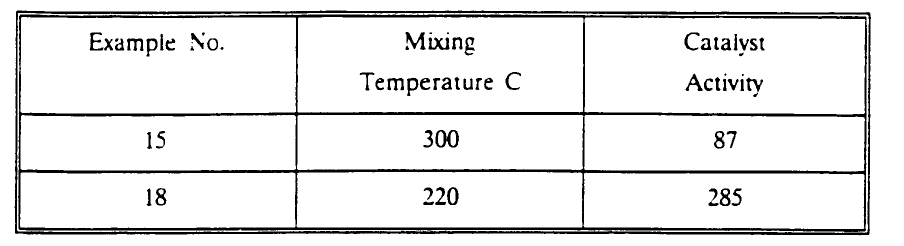

### Example 19 (Comparative)

This example shows the preparation of a catalyst prepared from an alumina with a surface area outside of the selected range. Alumina powder (30g) with a surface area of 200 m²/g and 4.5g sodium metal, cut into pieces, or placed into a round bottom flask under a nitrogen atmosphere at room temperature. The mixture was stirred and heated to 150°C for 30 minutes. During this step, the sodium metal was dispersed onto the alumina resulting in a bluish-gray powder. The stirring was continued while the temperature was maintained at 150°C for 60 minutes. The mixture was then cooled to room temperature. A gaseous stream of nitrogen containing 5 vol % oxygen was passed through the round bottom flask at a rate of 126 mL/min until the molar ratio of 0₂ to initial sodium was 0.2:1. The activity rate constant for this catalyst for isomerization of VNB to ENB at room temperature was measured as 15.

Comparison of the results from this Example 19 with the results of Example 12 shows that the temperature of mixing sodium and alumina leads to different catalytic activity even for catalysts with surface areas outside of the ranges preferred in the invention.

### Example 20

This example shows the preferred amounts of O₂ added to a catalyst during catalyst preparation. Figure 5 graphically shows the relationship between the activity rate constant and the ratio of O₂ to initial sodium.

In each of four separate preparations of catalysts, 30 g alumina powder with a surface area of 178 m²/g, and 4.5 g sodium metal, cut into pieces, were placed in a round bottom flask under a nitrogen atmosphere at room temperature. The mixture was stirred and heated to a temperature of 150°C for 30 minutes. After 30 minutes. the sodium metal was dispersed onto the alumina and the powder had changed in color to bluish-gray. The temperature of the mixture was then raised to 300°C for 60 minutes while stirring continued. The mixture was then cooled to room temperature. A nitrogen stream containing 5 vol % oxygen was then passed through the flask at 126 mL/min until the ratio of O₂ to initial sodium was 0.1:1. The catalyst preparation was repeated except that the nitrogen stream containing 5 vol % O₂ was passed through the flask until the ratio of O₂ to initial sodium was 0.2:1. A third catalyst was prepared in a similar manner so that the ratio of O₂ to initial sodium was 0.5:1. A fourth catalyst was prepared in the same manner except that no oxygen was added to the mixture of sodium dispersed on alumina. The results of an analytical test to determine the amount of unreacted sodium on the catalyst is set forth below in Table 2.

**Table 2**

| Sample No. | O₂/Na Ratio | % Unreacted Na Metal |
|---|---|---|
| 20-1 | 0.1:1 | 33 |
| 20-2 | 0.2:1 | 5 |
| 20-3 | 0.5:1 | 5 |
| 20-5 | 0:1 | 81 |

The isomerization activity of the four catalyst prepared above was measured and is set forth in Figure 5. These results show that the most preferred ratio of O₂ to sodium is 0.1:1.

As can be seen from the conversion levels above, the alumina with a surface area of about 180 m²/g performed better than aluminas with surface areas of 210 m²/g. Aluminas with surface areas of about 170 m²/g are most preferred.

## Claims

1. An activated catalyst for use in isomerization of olefins prepared by:
(a) heating a support material other than hydrotalcite to form a calcined support material having a surface area of 150 to 180 m²/g
(b) contacting the calcined support material with a metallic form alkali metal to disperse the alkali metal on the support material and form a catalyst precursor mixture of alkali metal and calcined support material; and
(c) contacting the mixture with an oxygen containing activating gas.

2. An activated catalyst according to Claim 1, wherein the support material in step (a) is alumina.

3. An activated catalyst according to Claims 1 or 2, wherein the alkali metal in step (b) is sodium, potassium, cesium or mixtures thereof, preferably sodium.

4. An activated catalyst according to any of the preceding claims, wherein the temperature at which the alkali metal is dispersed on the calcined support in step (b) is 100°C to 300°C.

5. An activated catalyst according to any of the preceding claims, wherein the catalyst precursor mixture is contacted with the activating gas in step (c) at a temperature of from 55°C to 300°C, preferably 100°C to 300°C.

6. An activated catalyst according to any of the preceding claims, wherein the activating gas in step (c) comprises nitrogen and from 2 to 10 mole percent oxygen.

7. An activated catalyst according to any of the preceding claims, wherein the ratio of total moles of activating oxygen to total atoms of alkali metal in step (c) is in the range of 0.005:1 to 1:1.

8. The use of an activated catalyst as prepared in accordance with any of the preceding claims, to isomerize an olefin or a stream containing an olefin, wherein the olefin or stream containing the olefin is contacted with the activated catalyst at a temperature of from 20°C to 100°C, preferably 100°C.

9. The use in accordance with Claim 8, wherein the olefin is an alkenyl bridged ring compound, preferably 5-vinyl-2-norbornene.

10. The use in accordance with Claim 9, wherein the stream containing the olefin further comprises an isomerization catalyst poison.

## Patentansprüche

1. Aktivierter Katalysator zur Verwendung bei der Isomerisierung von Olefinen, der hergestellt ist durch
(a) Erwärmen eines von Hydrotalcit verschiedenen Trägermaterials, um ein calciniertes Trägermaterial mit einer Oberfläche von 150 bis 180 m²/g zu bilden,
(b) Kontaktieren des calcinierten Trägermaterials mit einem Alkalimetall in metallischer Form, um das Alkalimetall auf dem Trägermaterial zu dispergieren und eine Katalysatorvorläufermischung aus Alkalimetall und calciniertem Trägermaterial zu bilden, und
(c) Kontaktieren der Mischung mit einem sauerstoffhaltigen Aktivierungsgas.

2. Aktivierter Katalysator nach Anspruch 1, bei dem das Trägermaterial in Stufe (a) Aluminiumoxid ist.

3. Aktivierter Katalysator nach Anspruch 1 oder 2, bei dem das Alkalimetall in Stufe (b) Natrium, Kalium, Cäsium oder Mischungen derselben und vorzugsweise Natrium ist.

4. Aktivierter Katalysator nach einem der vorhergehenden Ansprüche, bei dem die Temperatur, bei der das Alkalimetall auf dem calcinierten Träger in Stufe (b) dispergiert wird, 100°C bis 300°C beträgt.

5. Aktivierter Katalysator nach einem der vorhergehenden Ansprüche, bei dem die Katalysatorvorläufermischung mit dem Aktivierungsgas in Stufe (c) bei einer Temperatur von 55°C bis 300°C, vorzugsweise 100°C bis 300°C, kontaktiert wird.

6. Aktivierter Katalysator nach einem der vorhergehenden Ansprüche, bei dem das Aktivierungsgas in Stufe (c) Stickstoff und 2 bis 10 Mol.% Sauerstoff umfaßt.

7. Aktivierter Katalysator nach einem der vorhergehenden Ansprüche, bei dem das Verhältnis der Gesamtmole Aktivierungssauerstoff zu den Gesamtatomen Alkalimetall in Stufe (c) im Bereich von 0,005:1 bis 1:1 liegt.

8. Verwendung eines gemäß einem der vorhergehenden Ansprüche hergestellten aktivierten Katalysators zum Isomerisieren eines Olefins oder eines Olefin enthaltenden Stroms, wobei das Olefin oder der Olefin enthaltende Strom mit dem aktivierten Katalysator bei einer Temperatur von 20°C bis 100°C, vorzugsweise 100°C, kontaktiert wird.

9. Verwendung nach Anspruch 8, bei der das Olefin eine alkenylverbrückte Ringverbindung ist, vorzugsweise 5-Vinyl-2-norbornen.

10. Verwendung nach Anspruch 9, bei der der das Olefin enthaltende Strom außerdem ein Isomerisierungskatalysatorgift enthält.

## Revendications

1. Catalyseur activé destiné à être utilisé dans l'isomérisation d'oléfines, préparé :
(a) en chauffant une matière de support autre que l'hydrotalcite pour former une matière de support calcinée ayant une surface spécifique de 150 à 180 m²/g ;
(b) en mettant en contact la matière de support calcinée avec un métal alcalin sous forme métallique pour disperser le métal alcalin sur la matière de support et former un mélange précurseur de catalyseur constitué du métal alcalin et de la matière de support calcinée ; et
(c) en mettant en contact le mélange avec un gaz d'activation contenant de l'oxygène.

2. Catalyseur activé suivant la revendication 1, dans lequel la matière de support dans l'étape (a) consiste en alumine.

3. Catalyseur activé suivant la revendication 1 ou 2, dans lequel le métal alcalin dans l'étape (b) consiste en sodium, potassium, césium ou leurs mélanges, de préférence sodium.

4. Catalyseur activé suivant l'une quelconque des revendications précédentes, dans lequel la température à laquelle le métal alcalin est dispersée sur le support calciné de l'étape (b) est comprise dans l'intervalle de 100°C à 300°C.

5. Catalyseur activé suivant l'une quelconque des revendications précédentes, dans lequel le mélange précurseur de catalyseur est mis en contact avec le gaz d'activation dans l'étape (c) à une température comprise dans l'intervalle de 55°C à 300°C, de préférence de 100°C à 300°C.

6. Catalyseur activé suivant l'une quelconque des revendications précédentes, dans lequel le gaz d'activation dans l'étape (c) comprend l'azote et 2 à 10 moles pour cent d'oxygène.

7. Catalyseur activé suivant l'une quelconque des revendications précédentes, dans lequel le rapport du nombre total de moles d'oxygène d'activation au nombre total d'atomes de métal alcalin dans l'étape (c) est compris dans l'intervalle de 0,005:1 à 1:1.

8. Utilisation d'un catalyseur activé préparé suivant l'une quelconque des revendications précédentes pour l'isomérisation d'une oléfine ou d'un courant contenant une oléfine, dans laquelle l'oléfine ou le courant contenant l'oléfine est mis en contact avec le catalyseur activé à une température de 20°C à 100°C, de préférence de 100°C.

9. Utilisation suivant la revendication 8, dans laquelle l'oléfine est un composé cyclique à pontage alcényle, de préférence le 5-vinyl-2-norbornène.

10. Utilisation suivant la revendication 9, dans laquelle le courant contenant l'oléfine comprend en outre un poison de catalyseur d'isomérisation.
